# EUROPÄISCHE PATENTSCHRIFT

(11) **EP 3 746 150 B1**
(45) Veröffentlichungstag und Bekanntmachung des Hinweises auf die Patenterteilung: **06.03.2024**
(21) Anmeldenummer: 19702836.8
(22) Anmeldetag: 24.01.2019
(51) Int. Cl.: A61M 1/28, A61M 1/14

(54) **VORRICHTUNG ZUR BESTIMMUNG DES STATISCHEN PATIENTENDRUCKS**
APPARATUS TO DETERMINE THE STATISTICAL PATIENT PRESSURE
APPAREIL DE DÉTERMINATION DE LA PRESSION STATISTIQUE DU PATIENT

(30) Priorität: 31.01.2018 DE 102018102151
(43) Veröffentlichungstag der Anmeldung: 09.12.2020
(73) Patentinhaber: Fresenius Medical Care Deutschland GmbH, 61352 Bad Homburg (DE)
(72) Erfinder: HOCHREIN, Torsten, 91353 Hausen (DE); HEDMANN, Frank, 97332 Volkach (DE)
(74) Vertreter: Herrmann, Uwe
(86) Internationale Anmeldenummer: PCT/EP2019/051798
(87) Internationale Veröffentlichungsnummer: WO 2019/149620

(56) Entgegenhaltungen:
- EP-B1- 2 776 085
- WO-A1-2016/080883
- US-A1- 2012 310 148
- US-A1- 2015 057 601

## Beschreibung

Die vorliegende Erfindung betrifft eine Vorrichtung zur Bestimmung des statischen Patientendrucks. Eine herkömmliche Vorrichtung zur Bestimmung des statischen Patientendrucks eines Peritonealdialysepatienten ist z.B. aus der WO2016/080883A1 bekannt.

Während einer Peritoneal-Dialysebehandlung wird ein Volumen an Dialyseflüssigkeit über eine Patientenleitung in den Bauchraum eines Patienten eingeführt und dort für eine bestimmte Verweildauer belassen. Das Peritoneum wirkt als semipermeable Membran, die während der Verweildauer der Dialyseflüssigkeit in dem Bauchraum einen Stoffübergang von dem Blut in die Dialyseflüssigkeit ermöglicht, wodurch eine Blutreinigung erreicht wird.

Nach einer bestimmten Verweilzeit wird die Dialyseflüssigkeit aus dem Bauchraum vollständig oder teilweise durch die Patientenleitung abgelassen und es wird frische Dialyseflüssigkeit in den Bauchraum eingeführt.

Während des vollständigen Auslaufes, d.h. während des Abführens der Dialyselösung aus dem Bauchraum entsteht ein Unterdruck. Dieser in der Dialyselösung gemessene Druck setzt sich aus dem dynamischen Druck bedingt durch die Strömung der Dialyselösung, d.h. dem durch den Flusswiderstand bedingten Förderdruck, aus dem statischen Druck bedingt durch die Höhenlage des Patienten sowie aus dem Druck im Bauchraum, d.h. aus dem peritonealen Druck zusammen.

Der auf diese Weise gemessene Druck besteht somit aus der Summe der einzelnen Teildrücke, so dass es für das Dialysegerät bzw. für den Anwender nicht möglich ist, den gemessenen Gesamtdruck einzelnen Druckkomponenten zuzuordnen.

Um den statischen Druckanteil zu erfassen, ist es notwendig, dynamische Druckanteile zu eliminieren, d.h. die Pumpe zur Förderung der Dialyselösung anzuhalten. Der statische Druckanteil legt fest, wieviel Energie durch die Pumpe aufgebracht werden muss, um den Höhenunterschied zwischen dem Patienten und der Pumpe bzw. dem Dialysegerät zu überwinden.

Denkbar ist beispielsweise eine Vorgehensweise, bei der z.B. zu Beginn der Auslaufphase, d.h. zu Beginn der Entleerung des Bauchraums des Patienten, die Pumpe in eine Neutralstellung gefahren und dann angehalten wird und der Druck in der Pumpkammer ausgeregelt wird, so dass kein Überdruck in der Pumpkammer besteht. Im Anschluss daran wird ein Flussweg zwischen der Pumpe und der Patientenleitung hergestellt. Der sich nun einstellende Druck bildet die Patientenlage ab und stellt den statischen Druckanteil dar. Er spiegelt den Höhenunterschied zwischen dem Patienten und der Pumpe wieder.

Diese Annahme ist allerdings nur korrekt, wenn die Patientenleitung nicht teilweise oder vollständig verschlossen, wie z.B. abgeknickt ist. Wäre die Patientenleitung beispielsweise verschlossen, würde der Saugdruck einer vorherigen Auslaufphase den gemessenen Druckwert dahingehend verfälschen, dass ein zu geringer Druck gemessen wird. Das Dialysegerät würde aus dem geringen gemessenen statischen Druckwert auf eine zu niedrige Patientenlage schließen und demzufolge den Saugdruck bei der Entleerung des Patienten erhöhen, um die vermeintlich große Höhendifferenz zwischen Patient und Pumpe zu bewerkstelligen. Diese Erhöhung des Saugdrucks der Pumpe kann zu einer Gefährdung des Patienten führen.

Im Falle eines Verschlusses der Patientenleitung wird somit zwar ebenfalls ein Druckwert ermittelt, der jedoch nicht dem statischen Druckanteil entspricht, der Rückschlüsse auf die Höhenlage des Patienten zulässt.

Aus der WO 2016/080883 A1 ist es bekannt, durch Aufgabe eines erhöhten Messdruckes auf einen geknickten Schlauch zu schließen.

Der vorliegenden Erfindung liegt vor diesem Hintergrund die Aufgabe zugrunde, eine Vorrichtung der eingangs genannten Art dahingehend weiterzubilden, dass eine Bestimmung des korrekten statischen Patientendrucks möglich Ist.

Diese Aufgabe wird durch eine Vorrichtung mit den Merkmalen des Anspruchs 1 gelöst.

Die erfindungsgemäße Vorrichtung zur Bestimmung des statischen Patientendrucks, weist eine mit einer Patientenleitung in Verbindung stehende Pumpe zur Förderung einer Dialyselösung in den Bauchraum auf. Unter dem Begriff "Patientenleitung" ist die Leitung, Insbesondere die Schlauchleitung zu verstehen, die von der Vorrichtung zu dem Patienten führt. Der Begriff "Patientenleitung" kann den Patientenkatheter mit umfassen, d.h. den Leitungsabschnitt, der mit dem Patienten fest in Verbindung steht und In den Bauchraum des Patienten führt. Unter dem Begriff der Patientenleitung kann aber auch der Leitungsabschnitt bis zu dem Patientenkatheter verstanden werden.

Die Vorrichtung weist zumindest eine Messeinrichtung zum Messen des Druckes z.B. in der Dialyselösung auf. Dieser Drucksensor kann an jeder beliebigen Stelle der Vorrichtung angeordnet sein, an der eine Änderung des statischen Druckes der Dialyselösung gemessen werden kann. Er ist so angeordnet, dass er einen Druck bzw. eine Druckänderung misst, die sich ergibt, wenn das Absperrelement der Patientenleitung geöffnet wird.

Des Weiteren ist ein Absperrmittel vorgesehen, insbesondere ein Ventil, durch das die Patientenleitung absperrbar Ist.

Die Vorrichtung weist des Weiteren wenigstens eine Druckkammer auf, die mit der Patientenleitung bei geöffnetem Absperrelement in Fluidverbindung steht, sowie eine Steuereinheit, die derart ausgebildet ist, dass diese in einem ersten Schritt einen Druckaufbau in der Druckkammer auf einen Messdruck bewirkt und in einem zweiten Schritt das Absperrelement öffnet, so dass die Patientenleitung oder ein Teilabschnitt von dieser mit dem Messdruck der Druckkammer beaufschlagt wird. Während des Druckaufbaus ist das Absperrelement der Patientenleitung vorzugsweise geschlossen.

Der Drucksensor ist so angeordnet, dass er die Änderung des Druckes in der Druckkammer oder in einem damit in Fluidverbindung stehenden Element oder einen damit korrelierten Druck misst.

Die Auswerteeinheit der Vorrichtung erfasst den gemessenen Druck nach dem Öffnen des Absperrelementes und wertet diesen aus.

Ist die Patientenleitung frei, d.h. weder geknickt bzw. in Ihrem Innendurchmesser verringert noch ganz verschlossen, kann sich der In der Druckkammer aufgebaute Druck in die Patientenleitung und in den Bauchraum des Patienten ausbreiten bzw. ausgleichen. Es stellt sich der statische Patientendruck ein, der den Höhenunterschied zwischen dem Patienten und der Vorrichtung bzw. dem Perltonealdialysegerät wiederspiegelt. Auf diese Weise Ist es möglich, den statischen Patientendruck zu messen.

Ist die Patientenleitung jedoch verschlossen, kann sich der In der Druckkammer aufgebaute Messdruck nicht über die gesamte Patientenleitung bis in den Bauchraum hinein ausbreiten, was zur Folge hat, dass sich ein anderer gemessener Druckwert einstellt, als für den Fall einer freien Patientenleitung.

Ist die Patientenleitung geknickt bzw. weist diese einen verringerten Strömungsdurchmesser auf, so findet der Druckausgleich zwischen der Druckkammer und der Patientenleitung mit geringerer Geschwindigkeit statt als bei einer freien Patientenleitung, was durch die Auswerteeinheit ebenfalls erfasst werden kann.

So ist es möglich, über die Messung des Druckes und/oder des zeitlichen Druckverlaufs Rückschlüsse darauf zu ziehen, ob die Patientenleitung verschlossen ist. Ist die Patientenleitung vollständig verschlossen, stellt sich in vergleichsweise kurzer Zeit ein Druckwert ein, der höher Ist als der Druckwert, der sich bei einer freien Patientenleitung ergeben würde. Ist die Patientenleitung Im Strömungsquerschnitt verkleinert, jedoch nicht vollständig verschlossen, stellt sich ggf. der statische Druck ein, jedoch erst nach einer längeren Zeitspanne als bei einer freien Patientenleitung.

Vorzugsweise ist die Steuereinheit derart ausgebildet, dass der in der Druckkammer aufgebaute Messdruck größer ist, als der Saugdruck in der Patientenleitung nach dem Öffnen des Absperrelementes.

Die Druckkammer ist vorzugsweise Bestandteil der Pumpe, wie z.B. die Druckseite einer Membranpumpe. Sie kann aber auch an anderer Stelle, z.B. zwischen der Pumpe und der Patientenleitung angeordnet sein.

Bei der Druckkammer kann es sich um ein beliebiges Element handeln, in das die Dialyselösung gefördert werden kann und darin unter Druck gehalten werden kann, bis die Messung durchgeführt wird. Denkbar ist es, dass die Druckkammer durch einen Teil einer Disposable-Kassette gebildet wird.

Die Kassette kann Mittel zur Steuerung des Dialysatflusses und/oder Absperrmittel zur Unterbindung des Dialysatflusses aufweisen bzw. mit Aktuatoren zusammenwirken, die eine solche Flusssteuerung oder Absperrung bewirken. Vorzugsweise umfasst die Kassette auch die Pumpe und/oder die Druckkammer.

Die Auswerteeinheit kann ausgebildet sein, den nach dem Öffnen des Absperrelementes gemessenen Druck mit einem Referenzdruck zu vergleichen. In Abhängigkeit des gemessenen Drucks bzw. der Differenz zwischen dem gemessenen Druck und dem Referenzdruck kann zum einen darauf geschlossen werden, ob die Patientenleitung oder der Bauchraum verschlossen Ist. Zum anderen kann aus dem gemessen Druck bzw. aus der Differenz des gemessenen Drucks und dem Referenzdruck oder dem Messdruck ein Rückschluss darauf gezogen werden, an welcher Stelle die Patientenleitung verstopft bzw. genickt ist. In einer bevorzugten Ausgestaltung der Erfindung ist die Auswerteinheit so ausgebildet, dass sie eine oder beide Prüfungen vornehmen kann.

Je höher der gemessene Druck ist, d.h. je geringer die Differenz aus dem Initialen Messdruck in der Druckkammer und dem gemessenen Druck ist, desto näher befindet sich der Verschluss etc. der Patientenleitung an der Druckkammer.

Grundsätzlich kann zur Auswertung in der Auswerteeinheit der Absolutwert des gemessenen Drucks, dessen Verlauf über die Zeit oder dessen Differenz zu einem Referenzdruck oder der zeitliche Verlauf dieser Differenz herangezogen werden. Bei dem Referenzdruck kann es sich um einen beliebigen Druck handeln, wobei der Referenzdruck auch durch den Messdruck gebildet werden kann, der vor der Messung in der Druckkammer bereitgestellt wird.

Über- oder unterschreitet der gemessene Druck oder dessen Differenz zu einem Referenzdruck oder die Geschwindigkeit der Druckänderung einen Grenzwert oder erreicht diesen, kann je nach Festlegung des Referenzdruckes auf eine teilweise oder vollständig verstopfte bzw. blockierte Patientenleitung geschlossen werden.

Die Auswerteeinheit kann beispielsweise ausgebildet sein, auf eine verschlossene oder abgeknickte Patientenleitung zu schließen, wenn die Differenz zwischen dem in der Druckkammer aufgebauten Messdruck und dem nach dem Öffnen des Absperrelementes gemessenen Druckes und/oder wenn die Geschwindigkeit der Änderung des gemessenen Drucks einen Grenzwert nicht erreicht.

Denkbar ist es, dass die Auswerteeinheit derart ausgebildet Ist, dass diese den Saugdruck der Pumpe in Abhängigkeit des gemessenen Drucks oder des zeitlichen Verlaufs des gemessenen Drucks einstellt. Ist die Patientenleitung abgeknickt oder teilweise verstopft, muss die Pumpe eine höhere Saugleistung bereitstellen als bei einer freien, d.h. weder teilweise oder vollständig verstopften oder genickten Patientenleitung.

Vorzugsweise ist die Auswerteeinheit derart ausgebildet, dass diese einen optischen oder akustischen Alarm ausgibt, wenn die Auswertung ergibt, dass die Patientenleitung nicht frei ist.

Dies kann z.B. dadurch ermittelt werden, dass die Differenz zwischen dem in der Druckkammer aufgebauten Messdruck und dem nach dem Öffnen des Absperrelementes gemessenen Druckes und/oder die Geschwindigkeit der Änderung des gemessenen Drucks einen Grenzwert nicht erreicht.

Wie oben ausgeführt, kann die Auswerteeinheit derart ausgebildet sein, dass diese basierend dem gemessenen Druck oder dessen Änderungsgeschwindigkeit oder der jeweiligen Differenz zu einem Referenzwert bzw. zu einer Referenzgeschwindigkeit eine Lokalisierung dahingehend vornimmt, an welcher Stelle eine Verengung oder ein Verschluss der Patientenleitung vorliegt.

Somit ist nicht nur feststellbar, dass die Patientenleitung teilweise oder vollständig verstopft oder geknickt Ist, sondern es lässt sich lokalisieren, an welcher Stelle dies der Fall ist.

Die vorliegende Erfindung betrifft des Weiteren ein Peritonealdialysegerät umfassend wenigstens eine Vorrichtung gemäß einem der Ansprüche 1 bis 9.

Die vorliegende Offenbarung betrifft des Weiteren ein nicht beanspruchtes Messverfahren zur Bestimmung des statischen Patientendrucks, wobei das Verfahren die folgenden Schritte aufweist:
a. Aufbauen eines Messdrucks in einer Druckkammer, während die Patientenleitung von der Druckkammer durch ein Absperrmittel getrennt ist,
b. Öffnen des Absperrmittels und
c. Messung des Drucks und/oder der zeitlichen Druckänderung nach dem Öffnen des Absperrmittels und
d. Auswertung des gemessenen Drucks und/oder der Druckänderung gemäß Schritt c).

Durch die Auswertung gemäß Schritt d) lässt sich ermitteln, ob die Patientenleitung frei, verstopft oder in Ihrem freien Strömungsquerschnitt verringert ist.

Vorzugsweise wird das Verfahren mittels einer Vorrichtung nach einem der Ansprüche 1 bis 9 oder mittels eines Peritonealdialysegerätes nach Anspruch 10 durchgeführt.

Es kann auf eine verschlossene oder abgeknickte Patientenleitung geschlossen werden, wenn der gemessene Druck oder wenn die Differenz zwischen dem In der Druckkammer aufgebauten Messdruck und gemessenen Druck und/oder wenn die Geschwindigkeit der Änderung des gemessenen Drucks einen Grenzwert nicht übersteigt.

Um dem geänderten Strömungswiderstand bei einer z.B. teilweise verstopften Saugleitung Rechnung zu tragen, wird vorzugsweise der Saugdruck der Pumpe in Abhängigkeit des gemessenen Drucks oder des zeitlichen Verlaufs des gemessenen Drucks eingestellt.

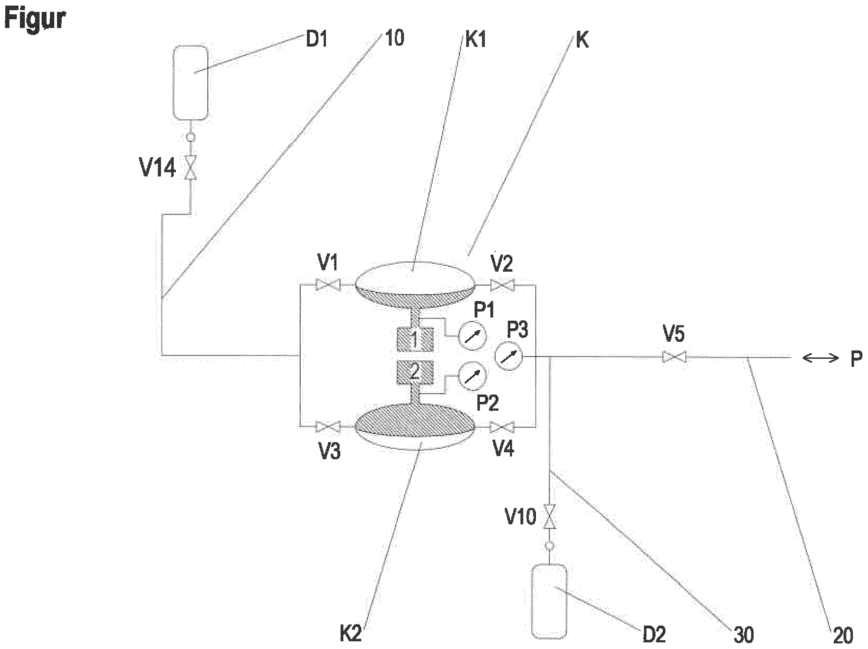

Weitere Einzelheiten und Vorteile der Erfindung werden anhand eines In der Zeichnung dargestellten Ausführungsbeispiels näher erläutert.

Die einzige Figur zeigt einen schematischen Flussplan eines Peritonealdialysegerätes gemäß der vorliegenden Erfindung.

Das Bezugszeichen D1 kennzeichnet einen Beutel oder sonstigen Behälter enthaltend frische Dialyselösung. Dieser steht mit der Leitung 10 in Fluidverbindung. Die Leitung 10 ist durch das Ventil V14 absperrbar und steht über eine Verzweigung mit den Saugleitungen der Membranpumpen 1 und 2 in Verbindung. Wie dies aus der Figur hervorgeht, befinden sich in den Saugleitungen der Pumpen Absperrventile V1 und V3.

Die Bezugszeichen P1 und P2 kennzeichnen Drucksensoren, die angeordnet sind, den jeweiligen Druck in dem Hydraulikmedlum der Membranpumpen 1 und 2 zu messen.

In den Druckleitungen der Pumpen 1 und 2 sind Absperrventile V2 und V4 angeordnet.

Die Ventile V1 bis V4 werden vorzugsweise so angesteuert, dass Im Betrieb des Gerätes die Pumpen 1 und 2 abwechselnd betrieben werden, so dass ein kontinuierlicher Fluss von Dialyselösung erzielt wird.

Das Bezugszeichen 20 kennzeichnet die Patientenleitung, in der sich das Absperrventil V5 befindet. Über die Patientenleitung 20 gelangt frisches Dialysat zum Patienten und gebrauchtes Dialysat vom Patienten P zu dem Drainagebeutel oder sonstigen Drainagebehälter D2. In der Drainageleitung 30 befindet sich das Ventil V10.

Die Pumpen 1, 2 weisen Druckkammern K1, K2 auf. Diese können Bestandteil einer Kassette sein kann, die als Disposable ausgeführt sein kann. Zwischen den Druckkammer K1, K2 und dem Absperrventil V5 befindet sich der Druckaufnehmer bzw. der Drucksensor P3.

An dieser Stelle wird darauf hingewiesen, dass der Drucksensor P3 optional ist. Grundsätzlich lässt sich die Erfindung auch nur mit dem oder den Drucksensoren P1 und/oder P2 realisieren.

Auch umgekehrt könnte die Druckmessung nur mit dem Drucksensor P3 stattfinden, so dass die Erfindung nur mit dessen Messwert und nicht unter Heranziehung der Druckwerte der Sensoren P1 und/oder P2 realisiert wird.

Auch Ist es von der Erfindung umfasst, dass die auf der Hydraulikseite (Druckmessung P1/P2) und kumulativ die auf der Dialysatseite (Druckmessung P3) ermittelten Werte im Rahmen der Erfindung verwendet.

Somit Ist sowohl eine alternative sowie auch eine kumulative Anordnung von Sensoren hydraulikseitig und/oder dialysatseitig bzw. deren Verwendung im Rahmen der vorliegenden Erfindung denkbar und von der Erfindung umfasst.

Vor der Einmessung des statischen Patientendrucks wird mittels der Pumpe 1 oder 2 oder mittels beider Pumpen 1, 2 ein erhöhter positiver Druck in der Druckkammer K1, K2 aufgebaut. Das Ventil V5 ist dabei geschlossen. Nach dem Aufbau des Drucks (Messdruck) in der Druckkammer K1, K2 wird das Ventil V5 in der Patientenleitung geöffnet und der Druck und/oder dessen zeitlicher Verlauf mittels des Drucksensors P3 gemessen.

Ist die Patientenleitung 20 frei, gleicht sich der aufgebaute Messdruck in die Patientenleitung 20 und in den Bauchraum des Patienten P aus. Es stellt sich der statische Patientendruck ein, der den Höhenunterschied von dem Patienten zu dem Dialysegerät wiederspiegelt.

Ist die Patientenleitung 20 jedoch nicht frei, sondern verschlossen, wird ggf. zwar auch eine Druckänderung gemessen, jedoch handelt es sich dabei um einen verfälschten Messwert, der dazu führen würde, dass eine zu hohe Patientenlage ermittelt wird, weil der Messdruck - wenn überhaupt - nur um einen relativ geringen Betrag fällt. Die Energiemenge der Pumpe wird reduziert.

Dieses Verhalten lässt den Saugdruck bei einer verschlossenen Patientenleitung ansteigen und führt zu einem sanfteren Anlaufverhalten.

Der genannte positive Messdruck in der Kammer K1, K2 ist so zu wählen, dass der anstehende Saugdruck überlagert wird. Dabei sind die Volumenverhältnisse des Schlauchsets zur Patientenleitung und der anstehende Saugdruck zu berücksichtigen.

Zum Aufbau des Messdrucks können eine oder beide der dargestellten Pumpen verwendet werden.

Ist der Patient noch mit Dialyselösung gefüllt, wenn das nicht beanspruchte Verfahren durchgeführt wird, erfolgt keine Volumenverschiebung, um das erfindungsgemäße Messverfahren durchzuführen. Ein separater Patientenleitungscheck ist nicht mehr notwendig, da sich das System selbst ausregelt. Grundsätzlich Ist die Erfindung jedoch auch in einem Zustand anwendbar, in dem der Patient teilweise oder vollständig entleert ist.

Ein bevorzugter Ablauf des nicht beanspruchten Verfahrens stellt sich wie folgt dar:

Das Peritonealdialysegerät befindet sich zu Beginn oder vor einer Auslaufphase, d.h. vor der Entleerung des Patienten.

Die Hydraulikpumpen 1, 2 werden in einen Pumpenbereich gefahren, der es erlaubt, in der Kammer K1 und/oder K2 einen Überdruck aufzubauen, ohne dem Einflussbereich der Pumpenmembran ausgesetzt zu sein.

Der Druck in der gesamten Kassette K wird in einen Überdruckbereich gehoben, z.B. auf einen Druckwert im Bereich von 50 mbar bis 400 mbar, vorzugsweise Im Bereich von 200 mbar bis 300 mbar und beispielsweise auf einen Druckwert von 250 mbar.

Es wird durch Öffnen des Ventils V5 ein Flussweg zwischen der Kassette K und der Patientenleitung 20 hergestellt und der Druck gemessen.

Dies kann über die hydraulikseitig angeordneten Drucksensoren P1 oder P2 erfolgen oder auch durch eine Druckmessung in der Dialyselösung, beispielsweise mittels des Drucksensors P3. Wie oben ausgeführt, kann die Druckmessung alternativ oder kumulativ mittels der hydraulikseitigen und/oder mittels der dialysatseitigen Drucksensoren erfolgen.

Der sich nun einstellende Druck bildet die Patientenlage ab, d.h. stellt den statischen Patientendruck dar.

Der ermittelte Wert wird einer nicht dargestellten Auswerteeinheit zugeführt.

Während des nicht beanspruchten Verfahrens sind das Ventil V10 der Leitung 30 sowie die Saugventile V1 und V3 geschlossen.

Ist die Patientenleitung 20 frei, wird sich der Druck in den Kammern K1, K2 über die Patientenleitung 20 bei geöffneten Ventilen V2, V4 und V5 in den Bauchraum des Patienten ausbreiten. Der Druck an den Sensoren P1, P2, P3 fällt.

Ist die Patientenleitung 20 nicht frei, sondern verstopft, wird sich der Druck bei geöffneten Ventilen V2, V4 und V5 In den Kammern K1, K2 nur bis zu der Knickstelle oder der Stelle der Verstopfung in die Patientenleitung 20 ausbreiten. Der Druck an den Sensoren P1, P2, P3 fällt ebenfalls, jedoch nur um einen geringeren Betrag als bei einer freien Patientenleitung.

Ist die Patientenleitung nicht vollständig verstopft bzw. abgeknickt, sondern nur der freie Strömungsquerschnitt, kommt es zu einer gegenüber einer freien Patientenleitung verlangsamten Druckverringerung.

Die Auswerteeinheit erfasst den Druckwert nach dem Öffnen des Ventils V5 bzw. den Druckverlauf nach dem Öffnen des Ventils. Ein Rechenalgorithmus der Auswerteeinheit bestimmt, ob die Patientenleitung teilweise oder vollständig blockiert bzw. verstopft Ist und wenn ja, wo dies der Fall ist.

Basierend auf der Auswertung der Auswerteeinheit kann die Leistung der Pumpen entsprechend angepasst werden, weil eine teilweise verstopfte Patientenleitung einen größeren Strömungswiderstand aufweist, als eine freie Patientenleitung und somit eine größere Saugleistung erfordert.

Ist die Patientenleitung teilweise oder vollständig verstopft, kann ein Alarm ausgegeben werden, der dies dem Anwender zur Kenntnis bringt.

## Patentansprüche

1. Vorrichtung zur Bestimmung des statischen Patientendrucks eines Peritonealdialysepatienten, wobei die Vorrichtung eine Patientenleitung (20), eine mit einer Patientenleitung (20) in Verbindung stehende Pumpe zur Förderung einer Dialyselösung in den Bauchraum des Patienten sowie Absperrmittel, insbesondere ein Ventil (V5), umfasst, durch das die Patientenleitung (20) absperrbar ist, wobei die Vorrichtung eine Druckkammer (K1) aufweist, die mit der Patientenleitung (20) bei geöffnetem Absperrelement in Fluidverbindung steht, wobei des Weiteren eine Druckmesseinrichtung (P1) vorgesehen ist, die derart angeordnet ist, dass diese den Druck in der Druckkammer (K1) oder einen mit diesem korrelierten Druck misst, wobei
die Vorrichtung eine Steuereinheit umfasst, die derart ausgebildet ist, dass diese in einem ersten Schritt einen Druckaufbau in der Druckkammer (K1) auf einen Messdruck bewirkt und in einem zweiten Schritt das Absperrelement öffnet, so dass die Patientenleitung (20) oder ein Teilabschnitt von dieser mit dem Messdruck der Druckkammer (K1) beaufschlagt wird und dadurch gezeichnet, dass
die Vorrichtung eine Auswerteeinheit aufweist, die dazu ausgelegt ist, den gemessenen Druck nach dem Öffnen des Absperrelementes auszuwerten, wobei die Auswerteeinheit derart ausgebildet ist, dass diese basierend auf der Differenz zwischen dem in der Druckkammer (K1) aufgebauten Messdruck und dem nach dem Öffnen des Absperrelementes gemessenen Druck und/oder basierend auf der Geschwindigkeit der Änderung des gemessenen Drucks eine Lokalisierung dahingehend vornimmt, an welcher Stelle eine Verengung oder ein Verschluss der Patientenleitung (20) vorliegt.

2. Vorrichtung nach Anspruch 1, **dadurch gekennzeichnet, dass** die Steuereinheit derart ausgebildet ist, dass der in der Druckkammer (K1) aufgebaute Messdruck größer ist, als der Saugdruck in der Patientenleitung (20) nach dem Öffnen des Absperrelementes.

3. Vorrichtung nach Anspruch 1 oder 2, **dadurch gekennzeichnet, dass** die Druckkammer (K1) zwischen der Pumpe und der Patientenleitung (20) angeordnet ist oder dass die Druckkammer (K1) einen Bestandteil der Pumpe bildet.

4. Vorrichtung nach einem der vorhergehenden Ansprüche, **dadurch gekennzeichnet, dass** die Druckkammer (K1) durch einen Teil einer Disposable-Kassette gebildet ist.

5. Vorrichtung nach Anspruch 4, **dadurch gekennzeichnet, dass** die Kassette die Pumpe und/oder die Druckkammer (K1) und/oder Mittel zur Steuerung des Dialysatflusses und/oder Absperrmittel zur Unterbindung des Dialysatflusses aufweist.

6. Vorrichtung nach einem der vorhergehenden Ansprüche, **dadurch gekennzeichnet, dass** die Auswerteeinheit ausgebildet ist, den nach dem Öffnen des Absperrelementes gemessenen Druck mit einem Referenzdruck oder mit dem Messdruck zu vergleichen und basierend darauf auf den Zustand der Patientenleitung zu schließen.

7. Vorrichtung nach einem der vorhergehenden Ansprüche, **dadurch gekennzeichnet, dass** die Auswerteeinheit ausgebildet ist, auf eine teilweise oder vollständig verschlossene Patientenleitung (20) zu schließen, wenn die Differenz zwischen dem in der Druckkammer aufgebauten Messdruck und dem nach dem Öffnen des Absperrelementes gemessenen Druck und/oder wenn die Geschwindigkeit der Änderung des gemessenen Drucks einen Grenzwert nicht übersteigt.

8. Vorrichtung nach einem der vorhergehenden Ansprüche, **dadurch gekennzeichnet, dass** die Auswerteeinheit derart ausgebildet ist, dass diese den Saugdruck der Pumpe in Abhängigkeit des gemessenen Drucks oder des zeitlichen Verlaufs des gemessenen Drucks einstellt.

9. Vorrichtung nach einem der vorhergehenden Ansprüche, **dadurch gekennzeichnet, dass** die Auswerteeinheit derart ausgebildet ist, dass diese einen Alarm ausgibt, wenn festgestellt wird, dass die Patientenleitung (20) nicht frei ist.

10. Peritonealdialysegerät umfassend wenigstens eine Vorrichtung gemäß einem der Ansprüche 1 bis 9.

## Claims

1. An apparatus for determining the static patient pressure of a peritoneal dialysis patient, wherein the apparatus comprises a patient line (20), a pump in communication with the patient line (20) for conveying a dialysis solution into the abdomen of the patient, and blocking means, in particular a valve (V5) by which the patient line (20) is blockable, wherein the apparatus comprises a pressure chamber (K1) that is in fluid communication with the patient line (20) with an open blocking element, wherein furthermore a pressure measurement device (P1) is provided that is arranged such that it measures the pressure in the pressure chamber (K1) or a pressure correlated with it; wherein the apparatus comprises a control unit that is configured such that it effects a pressure build-up in the pressure chamber (K1) to a measurement pressure in a first step and opens the blocking element in a second step so that the patient line (20) or a part section thereof is acted on by the measurement pressure of the pressure chamber (K1) and **characterized in that** the apparatus comprises an evaluation unit that is configured to evaluate the measured pressure after the opening of the blocking element, wherein the evaluation unit is configured such that it carries out a localization as to the position at which a constriction or a closure of the patient line (20) is present based on the difference between the measurement pressure (K1) built up in the pressure chamber and the pressure measured after opening the blocking element and/or based on the speed of the change of the measured pressure.

2. An apparatus in accordance with claim 1, **characterized in that** the control unit is configured such that the measurement pressure built up in the pressure chamber (K1) is larger than the suction pressure in the patient line (20) after the opening of the blocking element.

3. An apparatus in accordance with claim 1 or 2, **characterized in that** the pressure chamber (K1) is arranged between the pump and the patient line (20); or **in that** the pressure chamber (K1) forms a component of the pump.

4. An apparatus in accordance with one of the preceding claims, **characterized in that** the pressure chamber (K1) is formed by a part of a disposable cassette.

5. An apparatus in accordance with claim 4, **characterized in that** the cassette comprises the pump and/or the pressure chamber (K1) and/or means for controlling the dialysis flow and/or blocking means for suppressing the dialyzate flow.

6. An apparatus in accordance with one of the preceding claims, **characterized in that** the evaluation unit is configured to compare the pressure measured after the opening of the blocking element with a reference pressure or with the measurement pressure and, based on this, to draw a conclusion on the state of the patient line.

7. An apparatus in accordance with one of the preceding claims, **characterized in that** the evaluation unit is configured to draw a conclusion on a partly or completely closed patient line (20) when the difference between the measurement pressure built up in the pressure chamber and the pressure measured after the opening of the blocking element and/or when the speed of the change of the measured pressure does not exceed a limit value.

8. An apparatus in accordance with one of the preceding claims, **characterized in that** the evaluation unit is configured such that it sets the suction pressure of the pump in dependence on the measured pressure or on the time development of the measured pressure.

9. An apparatus in accordance with one of the preceding claims, **characterized in that** the evaluation unit is configured such that it outputs an alarm when it is found that the patient line (20) is not free.

10. A peritoneal dialysis machine comprising at least one apparatus in accordance with one of the claims 1 to 9.

## Revendications

1. Dispositif pour déterminer la pression de patient statique d'un patient en dialyse péritonéale, le dispositif comprenant une conduite de patient (20), une pompe communiquant avec une conduite de patient (20) pour transporter une solution de dialyse dans la cavité abdominale du patient ainsi que des moyens de blocage, notamment une soupape (V5), par lesquels la conduite de patient (20) peut être bloquée, le dispositif présentant une chambre de pression (K1) qui est en communication fluidique avec la conduite de patient (20) lorsque l'élément de blocage est ouvert, un appareil de mesure de pression (P1) étant en outre prévu, lequel est agencé de telle sorte que celui-ci mesure la pression dans la chambre de pression (K1) ou une pression corrélée à celle-ci, le dispositif comprenant une unité de commande, qui est configurée de telle sorte que celle-ci provoque, dans une première étape, une augmentation de pression dans la chambre de pression (K1) à une pression de mesure et, dans une deuxième étape, ouvre l'élément de blocage de telle sorte que la conduite de patient (20) ou une section partielle de celle-ci est soumise à la pression de mesure de la chambre de pression (K1) et **caractérisé en ce que** le dispositif présente une unité d'évaluation qui est conçue pour évaluer la pression mesurée après l'ouverture de l'élément de blocage, l'unité d'évaluation étant configurée de telle sorte que celle-ci procède, sur la base de la différence entre la pression de mesure établie dans la chambre de pression (K1) et la pression mesurée après l'ouverture de l'élément de blocage et/ou sur la base de la vitesse de variation de la pression mesurée, à une localisation en vue de déterminer à quel endroit il y a un rétrécissement ou une fermeture de la conduite de patient (20).

2. Dispositif selon la revendication 1, **caractérisé en ce que** l'unité de commande est configurée de telle sorte que la pression de mesure établie dans la chambre de pression (K1) est supérieure à la pression d'aspiration dans la conduite de patient (20) après l'ouverture de l'élément de blocage.

3. Dispositif selon la revendication 1 ou 2, **caractérisé en ce que** la chambre de pression (K1) est agencée entre la pompe et la conduite de patient (20) ou **en ce que** la chambre de pression (K1) forme un constituant de la pompe.

4. Dispositif selon l'une quelconque des revendications précédentes, **caractérisé en ce que** la chambre de pression (K1) est formée par une partie d'une cassette jetable.

5. Dispositif selon la revendication 4, **caractérisé en ce que** la cassette présente la pompe et/ou la chambre de pression (K1) et/ou des moyens de commande du débit de dialysat et/ou des moyens de blocage pour interrompre le débit de dialysat.

6. Dispositif selon l'une quelconque des revendications précédentes, **caractérisé en ce que** l'unité d'évaluation est configurée pour comparer la pression mesurée après l'ouverture de l'élément de blocage à une pression de référence ou à la pression de mesure et pour déduire l'état de la conduite de patient sur la base de cette comparaison.

7. Dispositif selon l'une quelconque des revendications précédentes, **caractérisé en ce que** l'unité d'évaluation est configurée pour conclure à une conduite de patient (20) partiellement ou totalement fermée lorsque la différence entre la pression de mesure établie dans la chambre de pression et la pression mesurée après l'ouverture de l'élément de blocage et/ou lorsque la vitesse de variation de la pression mesurée ne dépasse pas une valeur limite.

8. Dispositif selon l'une quelconque des revendications précédentes, **caractérisé en ce que** l'unité d'évaluation est configurée de telle sorte que celle-ci ajuste la pression d'aspiration de la pompe en fonction de la pression mesurée ou de l'évolution de la pression mesurée dans le temps.

9. Dispositif selon l'une quelconque des revendications précédentes, **caractérisé en ce que** l'unité d'évaluation est configurée de telle sorte que celle-ci émet une alarme lorsqu'il est constaté que la conduite de patient (20) n'est pas libre.

10. Appareil de dialyse péritonéale comprenant au moins un dispositif selon l'une quelconque des revendications 1 à 9.
